(19)

Europäisches Patentamt
European Patent Office
Office européen des brevets

(11) **EP 4 266 317 A1**

(12) **EUROPEAN PATENT APPLICATION**
published in accordance with Art. 153(4) EPC

(43) Date of publication:
**25.10.2023   Bulletin 2023/43**

(51) International Patent Classification (IPC):
*G16C 20/50* (2019.01)        *G16C 20/70* (2019.01)

(21) Application number: 22766169.1

(22) Date of filing: **28.02.2022**

(52) Cooperative Patent Classification (CPC):
**G16C 20/50; G06N 3/0455; G06N 3/047;
G06N 3/088;** G16C 20/70

(86) International application number:
**PCT/CN2022/078182**

(87) International publication number:
**WO 2022/188643 (15.09.2022 Gazette 2022/37)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  **10.03.2021  CN 202110260462**

(71) Applicant: **Tencent Technology (Shenzhen)
Company Limited
Shenzhen, Guangdong, 518057 (CN)**

(72) Inventors:
• **XU, Tingyang
Shenzhen, Guangdong 518057 (CN)**
• **HUANG, Junhong
Shenzhen, Guangdong 518057 (CN)**

• **XU, Shaoyong
Shenzhen, Guangdong 518057 (CN)**
• **TIAN, Li
Shenzhen, Guangdong 518057 (CN)**
• **CHEN, Xinde
Shenzhen, Guangdong 518057 (CN)**
• **LIU, Wei
Shenzhen, Guangdong 518057 (CN)**
• **HUANG, Junzhou
Shenzhen, Guangdong 518057 (CN)**
• **XUE, Ding
Shenzhen, Guangdong 518057 (CN)**
• **YU, Yang
Shenzhen, Guangdong 518057 (CN)**

(74) Representative: **Gunzelmann, Rainer
Wuesthoff & Wuesthoff
Patentanwälte PartG mbB
Schweigerstraße 2
81541 München (DE)**

(54) **METHOD AND APPARATUS FOR RECONSTRUCTING MOLECULAR STRUCTURE, AND
DEVICE, STORAGE MEDIUM AND PROGRAM PRODUCT**

(57)     Provided are a molecular structure reconstruction method and apparatus, a device, and a readable storage medium, relating to the field of machine learning. The method includes: 101: obtaining a set of structural data of a reference molecule, the reference molecule being a molecule that is active against a pre-set target, 102: performing structural separation on the obtained set of structural data to obtain a plurality of subsets of group data each corresponding to one of a plurality of molecular segment groups of the reference molecule, 103: performing feature processing on the group data of the molecular segment group to obtain a candidate segment for replacing the fragment segment, and 104: generating structural data of a reconstructed molecule based on the candidate segment and the side chain segment.

Obtain a set of structural data of a reference molecule, the reference molecule being a molecule that is active against a pre-set target — 101

Perform structural separation on the obtained set of structural data to obtain a plurality of subsets of group data each corresponding to one of a plurality of molecular segment groups of the reference molecule — 102

Perform feature analysis on the group data of the molecular segment group to obtain a candidate segment for replacing the fragment segment — 103

Generate structural data of a reconstructed molecule based on the candidate segment and the side chain segment — 104

**FIG. 1**

EP 4 266 317 A1

**Description**

RELATED APPLICATION

This application claims priority to Chinese Patent Application No. 202110260462.7, filed on March 10, 2021 and entitled "MOLECULAR STRUCTURE RECONSTRUCTION METHOD AND APPARATUS, DEVICE, STORAGE MEDIUM, AND PROGRAM PRODUCT", which is incorporated herein by reference in its entirety.

FIELD OF THE TECHNOLOGY

Embodiments of this application relate to the field of machine learning, and particularly to a molecular structure reconstruction method and apparatus, a device, a storage medium, and a program product.

BACKGROUND OF THE DISCLOSURE

Molecular generation refers to modifying an existing molecular structure to generate a molecular structure that differs from the existing molecular structure but maintains the same activity. For example, a reference molecule is a molecule that is active against a target, and then molecular generation is used to generate another structural molecule with the same activity at the target based on the reference molecule.

In the related art, molecular generation is performed by artificial intelligence (AI). The AI molecular generation technology generally generates a new structural molecule based on a reference molecule in combination with the molecular reconstruction ability and the molecular reconstruction validity.

However, during molecular reconstruction in the foregoing manner, a molecule is reconstructed based on rules, and it is difficult to get out of the main idea of a molecular structure design. As a result, it is unlikely to avoid an existing molecular structure for the reconstructed molecule, and the success rate of molecular reconstruction is low.

SUMMARY

The embodiments of this application provide a molecular structure reconstruction method and apparatus, a device, a storage medium, and a program product, which may improve the success rate and efficiency of molecular reconstruction. The technical solutions are as follows:

An aspect provides a molecular structure reconstruction method, applied a computer device, the method including:

obtaining a set of structural data of a reference molecule, the reference molecule being a molecule that is active against a pre-set target;

performing structural separation on the obtained set of structural data to obtain a plurality of subsets of group data each corresponding to one of a plurality of molecular segment groups of the reference molecule, the molecular segment groups comprising each a fragment segment of the reference molecule and a side chain segment corresponding to the fragment segment;

performing feature analysis on the group data of the molecular segment group to obtain a candidate segment for replacing the fragment segment; and

generating structural data of a reconstructed molecule based on the candidate segment and the side chain segment, the reconstructed molecule being active against the target.

Another aspect provides a molecular structure reconstruction apparatus, including:

an obtaining module, configured to obtain a set of structural data of a reference molecule, the reference molecule being a molecule that is active against a pre-set target;

a separation module, configured to perform structural separation on the obtained set of structural data to obtain a plurality of subsets of group data each corresponding to one of a plurality of molecular segment groups of the reference molecule, the molecular segment groups comprising each a fragment segment of the reference molecule and a side chain segment corresponding to the fragment segment; and

a generation module, configured to perform feature analysis on the group data of the molecular segment group to obtain a candidate segment for replacing the fragment segment,

the generation module being further configured to generate structural data of a reconstructed molecule based on the candidate segment and the side chain segment, the reconstructed molecule being active against the target.

Another aspect provides a computer device, including a processor and a memory, the memory storing at least one program, the at least one program being loaded and executed by the processor to implement the molecular structure reconstruction method according to any one of the embodiments of this application.

Another aspect provides a computer-readable storage medium, storing at least one instruction, at least one program, a code set, or an instruction set, the at least one instruction, the at least one program, the code set or the instruction set being loaded and executed by a processor to implement the molecular structure reconstruction method according to any one of the embodiments of this application.

Another aspect provides a computer program product or a computer program, including computer instructions, the computer instructions being stored in a computer-readable storage medium. A processor of a computer device reads the computer instructions from the computer-readable storage medium and executes the computer instructions to cause the computer device to perform the molecular structure reconstruction method according to any one of the embodiments.

Beneficial effects brought by the technical solutions provided in the embodiments of this application are at least as follows:

Structural separation is performed on structural data of a reference molecule to obtain a fragment segment and a side chain segment, so as to predict a new molecular segment structure replacing the fragment segment taking the side chain segment as a structure condition to obtain a candidate segment. That is, the molecule is separated, and a construction rule of a relevant segment is learned. The fragment segment on the original molecule is replaced with the newly generated candidate segment so as to implement the modification of the molecule. A relatively large number of candidate segments may be generated according to the structure condition, so that the probability that a newly generated molecule is different from an existing molecular structure is increased, and furthermore, the success rate of molecular reconstruction is increased.

BRIEF DESCRIPTION OF THE DRAWINGS

FIG. 1 is a flowchart of a molecular structure reconstruction method according to an exemplary embodiment of this application.

FIG. 2 is a schematic diagram of a scaffold separation process based on the embodiment shown in FIG. 1.

FIG. 3 is a schematic diagram of a scaffold separation result based on the embodiment shown in FIG. 1.

FIG. 4 is a schematic diagram of scaffold separation results based on the embodiment shown in FIG. 3.

FIG. 5 is a schematic diagram of fragment separation results based on the embodiment shown in FIG. 3.

FIG. 6 is a schematic diagram of a model training process according to an exemplary embodiment of this application.

FIG. 7 is a schematic diagram of a molecular reconstruction process according to an exemplary embodiment of this application.

FIG. 8 is a flowchart of a molecular structure reconstruction method according to another exemplary embodiment of this application.

FIG. 9 is a flowchart of a molecular structure reconstruction method according to another exemplary embodiment of this application.

FIG. 10 is a schematic diagram of a molecular site splicing manner based on the embodiment shown in FIG. 9.

FIG. 11 is an overall flowchart of a molecular structure reconstruction method according to an exemplary embodiment of this application.

FIG. 12 is a structural block diagram of a molecular structure reconstruction apparatus according to an exemplary embodiment of this application.

FIG. 13 is a structural block diagram of a molecular structure reconstruction apparatus according to another exemplary embodiment of this application.

FIG. 14 is a schematic structural diagram of a computer device according to an exemplary embodiment of this application.

DESCRIPTION OF EMBODIMENTS

Terms involved in the embodiments of this application are first introduced briefly.

Molecular reconstruction: it refers to modifying an existing molecular structure to generate a molecular structure that differs from the existing molecular structure but maintains the same activity. For example, a reference molecule is a molecule for a target. That is, the reference molecule is active against the target. Then, molecular generation is used to generate another structural molecule with the same activity at the target based on the reference molecule. Taking the field of medicine as an example, being active against the target means that a drug action may be generated at the target.

In the related art, an AI molecular generation technology is a technology for generating new structural molecules. The AI molecular generation technology needs to combine the molecular reconstruction ability and the molecular reconstruction validity based on a reference molecule, and the main concern is how to establish a Gaussian-distribution-based molecular hidden space. As a result, a molecular structure of a reconstructed molecule is very similar to that of a target, and it is unlikely to get out of a structural rule of the reference molecule.

The embodiments of this application provide an AI molecular design method for fragment modification of a reference molecule. The reference molecule is separated to obtain a fragment segment to be modified and replaced and a corresponding side chain segment. Then, after a new segment is generated based on the fragment segment, the side chain segment is spliced with the generated new segment to obtain a reconstructed molecular structure.

In the embodiments of this application, descriptions are made taking replacement of the fragment segment as an example. In some embodiments, the side chain segment may be replaced, or the side chain segment and the fragment segment are replaced. No limits are made thereto in the embodiments of this application.

It is to be noted that information (including, but not limited to, user equipment information, personal information of a user, etc.), data (including, but not limited to, data for analysis, stored data, displayed data, etc.), and signals involved in this application are authorized by the user or fully authorized by all parties, and the collection, use, and processing of relevant data need to comply with relevant laws and regulations and standards of relevant countries and regions. For example, reference molecules involved in this application are all obtained with full authority.

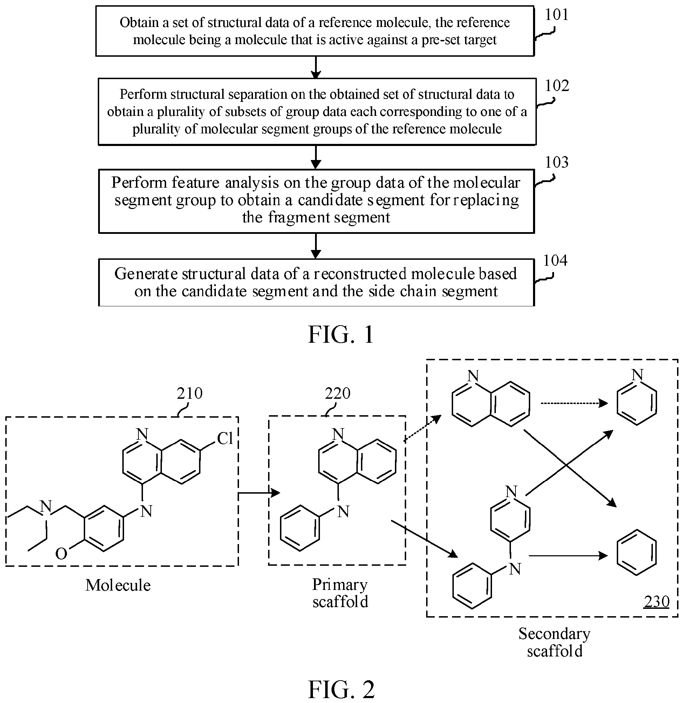

A molecular structure reconstruction method provided in the embodiments of this application is first described. FIG. 1 is a flowchart of a molecular structure reconstruction method according to an exemplary embodiment of this application. The method is described taking execution by a computer device (such as a terminal or a server) as an example. As shown in FIG. 1, the method includes the following steps:

Step 101: Obtain a set of structural data of a reference molecule, the reference molecule being a molecule that is active against a pre-set target.

In some embodiments, the reference molecule is a currently existing molecular structure. Optionally, the reference molecule is a protected molecular structure in a preset molecular library. Alternatively, the reference molecule is a molecule whose structure is to be simplified. Alternatively, the reference molecule is a molecule whose pharmacokinetic properties are to be improved.

In this embodiment, taking the field of medicine as an example, the reference molecule is a molecule for a target. That is, the reference molecule is active against the target, so as to generate a drug action at the target. In this embodiment of this application, structural reconstruction needs to be performed based on the reference molecule so as to generate a molecule that structurally differs from the reference molecule but has the same or similar activity. That is, the reconstructed molecule is also a molecule active against the target.

In some embodiments, the reference molecule is a molecular structure uploaded by a user through a molecular reconstruction interface. The user draws the molecular structure of the reference molecule on the molecular reconstruction interface, so that the computer device obtains the reference molecule. Alternatively, the user selects the stored molecular structure on the molecular reconstruction interface, so that the computer device obtains the reference molecule. Alternatively, the user edits and uploads a chemical formula of the reference molecule on the molecular reconstruction interface, so that the computer device obtains the reference molecule. The computer device may be a terminal itself or a server receiving the molecular structure uploaded by the terminal. No limits are made thereto in this embodiment.

In this embodiment of this application, the structural data of the reference molecule is implemented as a chemical formula of the reference molecule. Alternatively, the structural data of the reference molecule is implemented as a graphical structural representation of the reference molecule. No limits are made thereto in this embodiment.

Step 102: Perform structural separation on the obtained set of structural data to obtain a plurality of subsets of group data each corresponding to one of a plurality of molecular segment groups of the reference molecule,.

The reference molecule itself may have a plurality of (two or more) molecular segment groups. The molecular segment groups are obtained by dividing the reference molecule according to a certain preset rule. Each molecular segment group corresponds to its own group data. After the structure data of the reference molecule is split, the group data corresponding to the different molecular segment groups is obtained. The molecular segment groups include each a fragment segment of the reference molecule and a side chain segment corresponding to the fragment segment.

In some embodiments, structural separation needs to be performed on the structural data of the reference molecule based on a preset separation rule.

That is, a preset separation rule is obtained first, the preset separation rule including at least one of a scaffold separation rule and a rotatable bond separation rule. Structural separation is performed on the structural data of the reference molecule based on the preset separation rule to obtain group data of at least one molecular segment group corresponding to the reference molecule. A molecular segment group includes a fragment segment and at least one side chain segment. The fragment segment is obtained by separation according to the preset separation rule. After the fragment segment is deleted from the structural data of the reference molecule, a remaining structure is the side chain segment corresponding to the deleted fragment segment.

Since the preset separation rule includes at least one of the two separation rules, when a molecular segment groups are obtained by separation according to the scaffold separation rule, and b molecular segment groups are obtained by separation according to the rotatable bond separation rule, molecular reconstruction is performed through the a molecular segment groups, or molecular reconstruction is performed through the b molecular segment groups, or molecular reconstruction is performed through the a+b molecular segment groups. Here, a and b are both positive integers. In some embodiments, the generalization performance of a molecular reconstruction model is improved when molecular reconstruction is performed through the a+b molecular segment groups. The above-mentioned two preset separation rules are respectively described below.

**First: scaffold separation rule**

That is, the preset separation rule includes the scaffold separation rule. When structural separation is performed on the reference molecule, a scaffold structure meeting a scaffold requirement is extracted from the structural data of the reference molecule. The scaffold structure is deleted from the structural data of the reference molecule to obtain a side chain structure corresponding to the scaffold structure. The group data of the at least one molecular segment group corresponding to the reference molecule is obtained taking the scaffold structure as the fragment segment and the side chain structure as the side chain segment.

When the reference molecule is separated according to the scaffold separation rule, a corresponding primary scaffold is extracted first from the structural data of the reference molecule, and on the basis of obtaining the primary scaffold, a smaller secondary scaffold structure is obtained by subdivision according to different ring structures. The fragment segment obtained by separation based on the scaffold separation rule may be a primary scaffold structure in the reference molecule, or a secondary scaffold structure obtained by separation based on the primary scaffold structure.

Schematically, referring to FIG. 2, FIG. 2 is a schematic diagram of a scaffold separation process according to an exemplary embodiment of this application. As shown in FIG. 2, scaffold separation is performed first on a graphical structure of a reference molecule 210 to obtain a primary scaffold 220, and then separation is performed once or for many times based on the primary scaffold 220 to obtain a smaller secondary scaffold structure 230.

After the primary scaffold 220 and each secondary scaffold 230 are obtained, a scaffold structure meeting a scaffold requirement is selected from the primary scaffold 220 and the secondary scaffold 230 as a scaffold structure for separating the reference molecule. In some embodiments, when there are multiple scaffold structures meeting the scaffold requirement, after one of the scaffold structures meeting the scaffold requirement is deleted from the reference molecule, the reference molecule may be fragmented into one or more side chains, and the deleted scaffold structure and the side chains obtained by fragmentation are taken as a group to obtain a molecular segment group. That is, a molecular segment group includes a scaffold structure and at least one side chain.

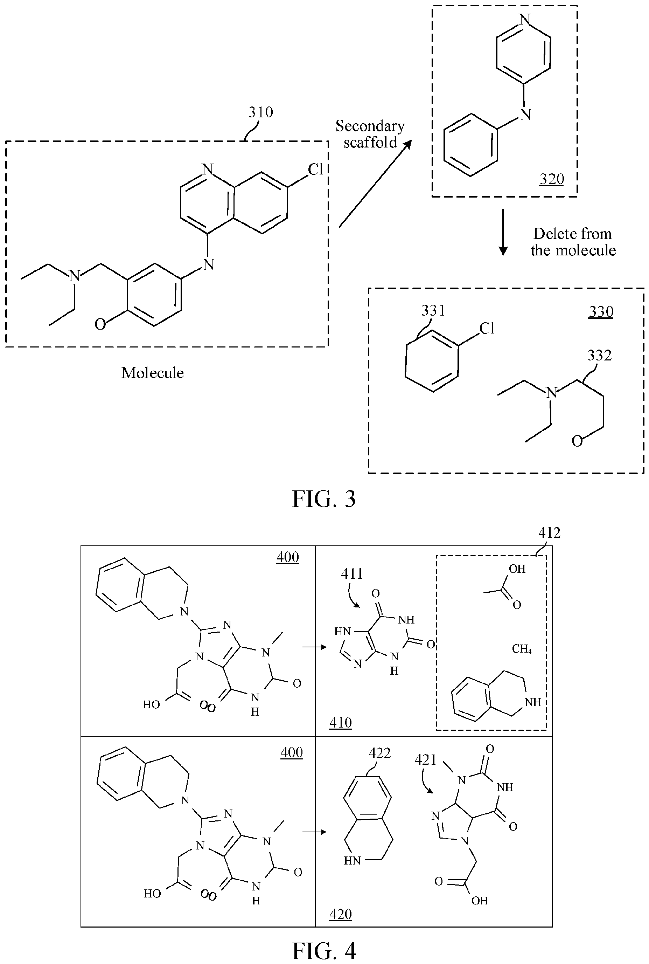

After the above-mentioned processing is performed on each scaffold structure meeting the scaffold requirement, at least one molecular segment group is obtained. In some embodiments, if at least two side chains are obtained after the scaffold structure is deleted, the scaffold structure and the at least two side chains are taken as a molecular segment group. Schematically, referring to FIG. 3, two side chains 330 are obtained after a secondary scaffold 320 is deleted from a molecule 310, including a side chain 331 and a side chain 332. A secondary scaffold 320 and the two side chains 330 are taken as a molecular segment group corresponding to the molecule 310.

In some embodiments, the group data of the molecular segment group includes the scaffold structure and the side chain segment corresponding to the scaffold structure. In some other embodiments, the group data of the molecular segment group may include the structural data of the reference molecule and the scaffold structure, and a molecular reconstruction model may directly determine the side chain segment according to the structural data of the reference molecule and the scaffold structure.

The scaffold requirement includes at least one of a ring number requirement, a heavy atom number requirement, and a rotatable bond number requirement. The scaffold structure whose ring number is within a range of the ring number requirement is extracted from the reference molecule in response to the scaffold requirement including the ring number requirement. The scaffold structure whose heavy atom number is within a range of the heavy atom number requirement is extracted from the reference molecule in response to the scaffold requirement including the heavy atom number requirement. The scaffold structure whose rotatable bond number is within a range of the requirement is extracted from the reference molecule in response to the scaffold requirement including the rotatable bond number requirement.

Schematically, the scaffold requirement specifically includes the following.

1: The number of rings in the scaffold structure is greater than or equal to 2. That is, the number of rings in the scaffold structure separated from the reference molecule needs to be greater than or equal to 2.

2: The number of heavy atoms in the scaffold structure is less than 20. That is, the number of heavy atoms in the scaffold structure separated from the reference molecule needs to be less than 20.

3: The number of rotatable bonds in the scaffold structure is less than 3. That is, the number of rotatable bonds in the scaffold structure separated from the reference molecule needs to be less than 3.

Schematically, referring to FIG. 4, FIG. 4 is a schematic diagram of a scaffold separation result according to an exemplary embodiment of this application. As shown in FIG. 4, there are at least two separation results after a reference molecule 400 is separated. In the first separation result 410, the reference molecule 400 is separated to obtain a scaffold structure 411 and a side chain 412. In the second separation result 420, the reference molecule 400 is separated to obtain a scaffold structure 421 and a side chain 422. In such case, the scaffold structure 411 and the side chain 412 form a molecular segment group, and the scaffold structure 421 and the side chain 422 form a molecular segment group.

**Second: rotatable bond separation rule**

That is, the preset separation rule also includes the rotatable bond separation rule. In such case, when structural separation is performed on the structural data of the reference molecule, the structural data of the reference molecule is cleaved from a rotatable bond to obtain the fragment segment meeting a fragment requirement, and the at least one molecular segment group corresponding to the reference molecule is obtained based on the fragment segment.

When the reference molecule is cleaved from the rotatable bond, a fragment obtained by cleaving needs to meet the fragment requirement. In some embodiments, the fragment requirement includes at least one of a fragment base requirement and a fragment scaffold requirement. When the reference molecule is cleaved from the rotatable bond, portions obtained by cleaving are referred to as fragments, and each fragment includes a scaffold. The fragment base requirement refers to a requirement for the whole fragment. The fragment scaffold requirement refers to a requirement for the scaffold in the fragment.

In some embodiments, the fragment base requirement includes at least one of a fragment ring number requirement, a fragment atom number requirement, a fragment rotatable bond number requirement, and a fragment structure requirement. The fragment scaffold requirement includes at least one of a scaffold atom number requirement and scaffold rotatable bond number requirement for the fragment scaffold.

Schematically, the fragment requirement specifically includes the following.

1: The fragment segment includes only one ring.

2: The number of non-H atoms in the fragment segment is greater than 5 and less than 30. That is, the number of non-hydrogen atoms in the fragment segment ranges from 5 to 30.

3: A size of the ring in the fragment segment is less than 8. It indicates that the ring in the fragment segment is a ring less than 8.

4: The number of rotatable bonds in the fragment segment is less than 9. It indicates that the number of rotatable bonds in the fragment segment is less than 9.

5: There is a fragment scaffold in the fragment segment. It indicates that the fragment segment is a segment including a scaffold.

6: The number of non-H atoms in the fragment scaffold is less than 20. It indicates that the number of non-hydrogen atoms in the scaffold of the fragment segment is less than 20.

7: The number of rotatable bonds in the fragment scaffold is less than 3. It indicates that the number of rotatable bonds in the scaffold in the fragment segment is less than 3.

8: A difference between the number of non-H atoms in the fragment segment and the number of non-H atoms in the fragment scaffold is less than 10.

9: The fragment segment includes more than one single-ring structure.

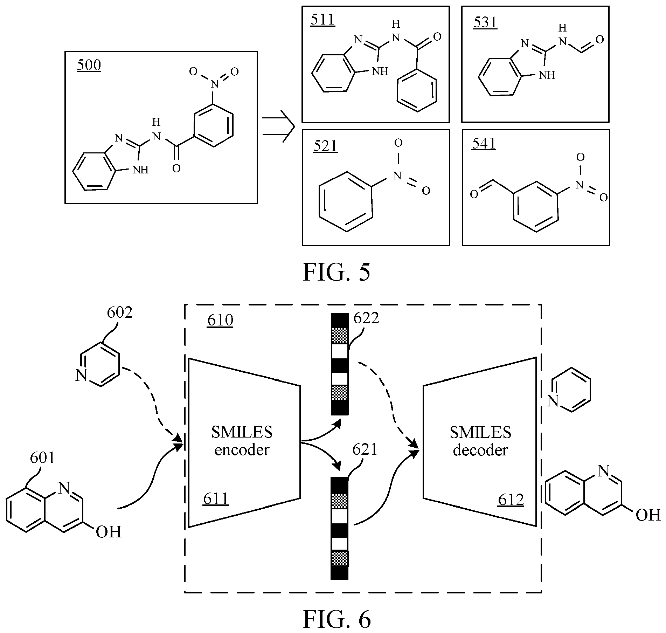

Schematically, referring to FIG. 5, FIG. 5 is a schematic diagram of fragment separation results according to an exemplary embodiment of this application. As shown in FIG. 5, there are at least four separation results after a graphical structure of a reference molecule 500 is separated. In the first separation result, the reference molecule 500 is separated to obtain a fragment segment 511. In the second separation result, the reference molecule 500 is separated to obtain a fragment segment 521. In the third separation result, the reference molecule 500 is separated to obtain a fragment segment 531. In the fourth separation result, the reference molecule 500 is separated to obtain a fragment segment 541. A side chain segment is obtained after the fragment segment obtained in each separation manner is deleted from the reference molecule. Each separation manner corresponds to a molecular segment group.

Step 103: Perform feature analysis on the group data of the molecular segment group to obtain a candidate segment for replacing the fragment segment.

In some embodiments, the group data of the molecular segment group is input to a molecular reconstruction model, and then feature analysis is performed on the group data of the molecular segment group through the molecular reconstruction model to obtain a candidate segment for replacing the fragment segment. Optionally, there is at least one molecular segment group for a reference molecule. In such case, group data of the at least one molecular segment group is sequentially input to the molecular reconstruction model, and then the molecular reconstruction model sequentially analyzes each of the at least one molecular segment group to obtain multiple candidate segments corresponding to each molecular segment group.

In some embodiments, the group data of the molecular segment group is encoded based on the side chain segment to generate a segment feature, and the segment feature is decoded to generate the candidate segment for replacing the fragment segment.

The molecular reconstruction model includes an encoder and decoder in an encoding and decoding architecture. The fragment segment is encoded first through the encoder based on the side chain segment to obtain a segment feature. A perturbation is applied to the segment feature through a preset perturbation rule to obtain a perturbed feature. Then, the perturbed feature is decoded through the decoder to generate the candidate segment for replacing the fragment segment. The perturbation is applied to improve the diversity of the generated candidate segment. That is, the perturbation is applied to modify the candidate segment based on the initial fragment segment and determine a candidate segment in the vicinity of a feature space corresponding to the initial fragment segment, so as to improve the diversity of the candidate segment.

When the segment feature is obtained by encoding, the side chain segment is mapped to a feature space to obtain a side chain spatial feature, and then the fragment segment is encoded through the encoder taking the side chain spatial feature as a structure condition to obtain the segment feature. In some embodiments, the fragment segment is encoded through the encoder to obtain an encoded feature first, and the encoded feature is mapped to the feature space taking the side chain spatial feature as the structure condition to obtain the segment feature.

The encoder, the decoder, and the feature space are obtained by pre-training. That is, mapping of the fragment segment and the side chain segment in the feature space is implemented according to the pre-trained molecular reconstruction model. The feature space is implemented as a Gaussian space satisfying a Gaussian distribution condition. The training and application of the molecular reconstruction models will be specifically described in the following embodiments.

Step 104: Generate structural data of a reconstructed molecule based on the candidate segment and the side chain segment.

In some embodiments, a hydrogen atom position on the candidate segment is spliced with the side chain segment to obtain structural data of a reconstructed molecule. The reconstructed molecule is active against the target. That is, the reconstructed molecule and the reference molecule are active against the same target.

It is also necessary to perform molecular structure screening on the reconstructed molecule obtained by splicing the candidate segment with the side chain segment through a preset screening process.

The candidate segment, side chain segment, fragment segment, candidate molecule, and the like involved in this embodiment are used for indicating structural data of the candidate segment, structural data of the side chain segment, structural data of the fragment segment, and structural data of the candidate molecule.

In summary, according to the molecular structure reconstruction method provided in this embodiment of this application, structural separation is performed on structural data of a reference molecule to obtain a fragment segment and a side chain segment, so as to predict a new molecular segment structure replacing the fragment segment taking the side chain segment as a structure condition to obtain a candidate segment. That is, the molecule is separated, and a construction rule of a relevant segment is learned. The fragment segment on the original molecule is replaced with the newly generated candidate segment so as to implement the modification of the molecule. A relatively large number of candidate segments may be generated according to the structure condition, so that the probability that a newly generated molecule is different from an existing molecular structure is increased, and furthermore, the success rate of molecular reconstruction is increased.

For the molecular reconstruction model in step 103, a training process and application process of the molecular reconstruction model are described respectively. The molecular structure of either a sample molecule in the model training process or the reference molecule in actual reconstruction needs to be separated into a fragment segment and a side chain segment. Model processing in the training process and the application process is respectively described as follows.

(1) The training process

The training process includes a pre-training process and a fine adjustment process.

In the pre-training process, molecular structures in a public database are mainly learned, so that the model may learn vector expressions of molecules. That is, the pre-training process is a model learning process of converting molecules into vector features.

In some embodiments, the candidate segment is predicted by the molecular reconstruction model.

Schematically, referring to FIG. 6, FIG. 6 is a schematic diagram of a model training process according to an exemplary embodiment of this application. Taking one molecular segment group obtained by separating a sample molecule as an example, as shown in FIG. 6, structural data of the sample molecule is separated to obtain a fragment segment 601 and a side chain segment 602. A molecular reconstruction model 610 includes an encoder 611 and a decoder 612. The encoder 611 encodes the fragment segment 601 and the side chain segment 602 to obtain a first segment vector 621 corresponding to the fragment segment 601 and a second segment vector 622 corresponding to the side chain segment 602. After the decoder 612 decodes the first segment vector 621 and the second segment vector 622, the encoder and decoder are trained with a purpose of recovering the fragment segment 601 and the side chain segment 602.

In this embodiment of this application, the molecular reconstruction model is improved based on a conditional variational autoencoder (CVAE). In the related art, a training condition parameter of the CVAE is a quantized vector. In this embodiment of this application, a molecule is separated into a fragment and a side chain, and then the side chain is input to the model as a structure condition. Specifically, after the side chain structure is mapped to an embedding space, the mapped structure embedding is used as a condition input. A specific optimization conditions refers to the following formula 1:

$$ELBO\left(\theta,\Theta;x,y\right) = \log p_\theta\left(x|f(y)\right) - KL\left[q_\Theta\left(z|x,f(y)\right)\|p_\theta\left(z|x,f(y)\right)\right]$$

$$\leqslant \log p_\theta\left(x|f(y)\right) - KL\left[q_\Theta\left(z|x,f(y)\right)\|p_\theta\left(z|f(y)\right)\right]$$

where $f(\cdot)$ represents a simplified molecular input line entry system (SMILES) encoder in this embodiment of this application. In some embodiments, the SMILES encoder initially shares parameters with the CVAE. By $f(\cdot)$, side chain y is mapped to the embedding space and subjected to SMILES decoding calculation with fragment x. The relative entropy (KL) represents discrete degree calculation. $\theta$ and $\Theta$ represent model parameters, i.e., parameters to be optimized during training. $p_\theta(x|f(y))$ represents a conditional probability distribution based on a condition of $f(y)$. $KL[q_\Theta(z|x,f(y))\|p_\theta(z|x,f(y))]$ represents a discrete degree between a $q_\Theta$ Gaussian distribution and a $p_\theta$ Gaussian distribution. z represents a feature space for feature mapping. The model parameters are trained by approximating the $q_\Theta$ Gaussian distribution and the $p_\theta$ Gaussian distribution.

After the structural data of the sample molecule is input to the molecular reconstruction model 610, a model parameter is trained according to a discrete degree between an output molecular segment and the fragment segment of the sample molecule, so as to optimize vector expressions extracted by the molecular reconstruction model 610 from a molecular structure, i.e., the first segment vector 621 and the second segment vector 622,, namely optimizing the encoder, and optimize a process of converting the vector expression into the molecular segment, namely optimizing the decoder.

The sample molecule used in the pre-training process is a molecule free of target constraints. Alternatively, the sample molecule used in the pre-training process is a molecule that is not labeled target information.

In the fine adjustment process, targeted training is performed based on the target. That is, a molecule active against the target are input and separated, and the model is further trained, so that a molecule reconstructed by the molecular reconstruction model 610 is more likely to be a molecule similar to the molecule active against the target. An adjustment manner for the model parameter in the fine adjustment process is the same as that for the model parameter in the pre-training process, and the difference lies in that the public database is changed to a molecular dataset active against the target.

(2) The application process

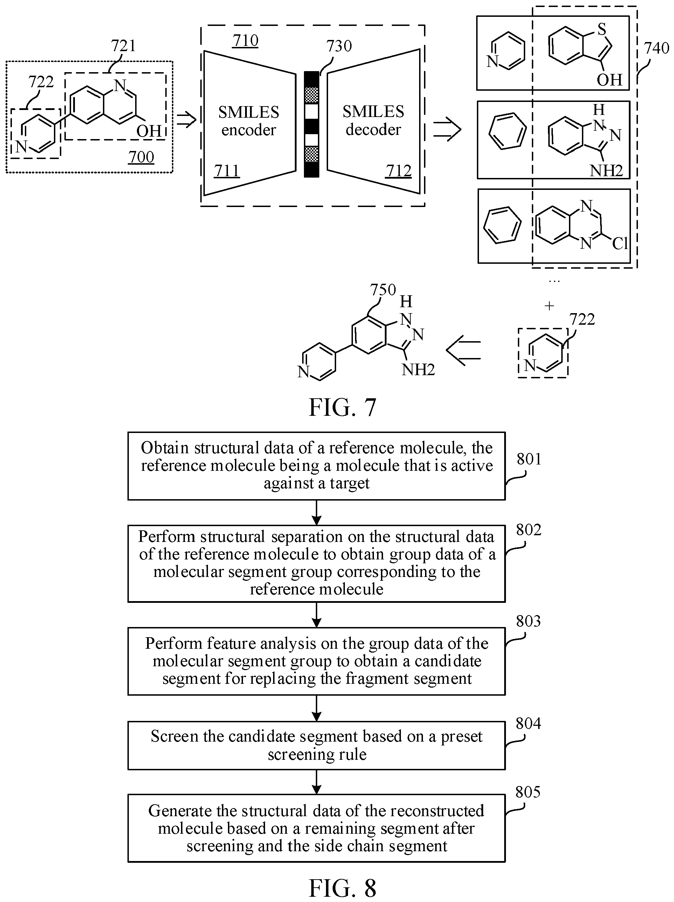

Schematically, referring to FIG. 7, FIG. 7 is a schematic diagram of a molecular reconstruction process according to this application. As shown in FIG. 7, structural data of a reference molecule 700 is first input to a trained molecular reconstruction model 710. The reference molecule 700 includes a fragment segment 721 and a side chain segment 722. Before being input to the molecular reconstruction model 710, the structural data of the reference molecule 700 is separated. Alternatively, the reference molecule 700 is separated by the molecular reconstruction model 710. Then, an encoder 711 encodes the fragment segment 721 in the reference molecule 700 to obtain a segment vector 730 corresponding to the fragment segment 721, and a decoder 712 decodes the segment vector 730 to obtain a candidate segment 740 for replacing the fragment segment 721. When the encoder 711 encodes the reference molecule 700, feature mapping of the fragment segment 721 is restricted taking the side chain segment 722 as a structure condition, so as to obtain the segment vector 730. It is necessary to further apply a perturbation to the segment vector 730 through a preset perturbation rule, so as to implement segment structure modification. A perturbed vector is determined in the vicinity of the segment vector 730 in a Gaussian space. Schematically, a feature value of the segment vector 730 is adjusted within a preset perturbation range. The side chain segment 722 is spliced with the candidate segment 740 to obtain structural data of reconstructed molecule 750.

In some embodiments, the application process further includes a screening process of the candidate segment 740 as well as a screening process of the reconstructed molecule 750, which will be described in the following embodiments.

The molecular reconstruction model may also be implemented as a natural language processing-based model, or a graph-structure-based model, etc. A specific implementation mode of the model is not limited in this embodiment of this application.

In some embodiments, the perturbation applied to the segment vector is executed in the application process, and no perturbations are applied to the segment vector in the training process of the molecular reconstruction model.

In some embodiments, after the molecular reconstruction model predicts the candidate segment, the candidate segment further needs to be screened. FIG. 8 is a flowchart of a molecular structure reconstruction method according to another exemplary embodiment of this application. The method is described taking execution by a computer device as an example. As shown in FIG. 8, the method includes the following steps:

Step 801: Obtain structural data of a reference molecule, the reference molecule being a molecule that is active against a target.

In this embodiment, taking the field of medicine as an example, the reference molecule is a molecule for a target. That is, the reference molecule is active against the target, so as to generate a drug action at the target.

Step 802: Perform structural separation on the structural data of the reference molecule to obtain group data of a molecular segment group corresponding to the reference molecule.

In some embodiments, structural separation needs to be performed on the reference molecule based on a preset separation rule.

That is, a preset separation rule is obtained first, the preset separation rule including at least one of a scaffold separation rule and a rotatable bond separation rule. A separation manner corresponding to the preset separation rule has been described in detail in step 102, and will not be elaborated herein.

Step 803: Perform feature analysis on the group data of the molecular segment group to obtain a candidate segment for replacing the fragment segment.

After the group data of the molecular segment group is input to a molecular reconstruction model, an encoder in the molecular reconstruction model encodes a fragment segment in the molecular segment group taking an encoded feature of a side chain segment as a structure condition to obtain an encoded vector. After the encoded vector is perturbed, a decoder in the molecular reconstruction model decodes the encoded vector to obtain a candidate segment for replacing the fragment segment.

Step 804: Screen the candidate segment based on a preset screening rule.

In some embodiments, the preset screening rule includes at least one of a validity screening rule, a uniqueness screening rule, a heavy atom number screening rule, and a ring screening rule.

The validity screening rule refers to retaining candidate segments that comply with SMILES validity and deleting candidate segments that do not comply with SMILES validity. SMILES is a specification for explicitly describing molecular structures with American standard code for information interchange (ASCII) character strings. That is, the generated candidate segment needs to comply with SMILES. In some embodiments, SMILES specifies grammatical rules of molecular structures. Schematically, a tetravalent nitrogen (N) atom position spliced with five bonds does not comply with SMILES.

The uniqueness screening rule refers to deduplicating repeated segments in multiple candidate segments to ensure that there is no duplication between each candidate segment and the other candidate segments. In some embodiments, since one or more candidate segments may be obtained based on a single molecular segment group, when multiple candidate segments may be obtained based on a single molecular segment group, the uniqueness screening rule is executed for candidate segments predicted based on one molecular segment group, or, for candidate segments predicted based on all molecular segment groups.

The heavy atom number screening rule refers to selecting a heavy atom number range according to the molecule to be reconstructed so as to select the generated candidate segment. Generally, the heavy atom number range is determined according to the heavy atom number of the reference molecule. Schematically, the reference molecule includes 12 heavy atoms. In such case, in some embodiments, the heavy atom number range of the molecule to be reconstructed is 9 to 15. Candidate segments are screened according to a requirement of the heavy atom number range to filter out the candidate segments whose heavy atom numbers do not comply with the heavy atom number range.

In some embodiments, it is also necessary to screen the candidate segment through a ring screening rule. Schematically, after the foregoing screening ends, candidate segments in a generated segment set are optionally further screened, including retaining only segments containing rings or aromatic rings. Optionally, the ring screening rule further includes a ring number requirement, or an aromatic ring number requirement, etc.

Step 805: Generate structural data of a reconstructed molecule based on a remaining segment after screening and a side chain segment.

The reconstructed molecule is a newly generated molecule active against the target. In some embodiments, since molecules of similar structures are more likely to be active against the same target, it is also necessary to obtain a segment similarity between the remaining segment and the fragment segment, retain q remaining segments with a maximum segment similarity, and generate the reconstructed molecule together with the side chain segment, q being a positive integer.

The segment similarity refers to a similarity between the remaining segment and the fragment segment in terms of 3-dimension (3D) structure. That is, after generated candidate segments are obtained and screened to obtain a large number of remaining segments, it is necessary to ensure that the remaining segments are close to a portion to be modified (i.e., the fragment segment portion) in the reference molecule, so as to keep the molecule active. In some embodiments, 3D similarities between the candidate segments (i.e., the remaining segments after screening) and a reference segment (i.e., the fragment segment in the reference molecule) are calculated using a chemical information software package RDKit or other 3D similarity calculation tools, and q segments with the maximum similarity are extracted and further spliced to finally obtain the reconstructed molecule.

In summary, according to the molecular structure reconstruction method provided in this embodiment of this application, structural separation is performed on a reference molecule to obtain a fragment segment and a side chain segment, so as to predict a new molecular segment structure replacing the fragment segment taking the side chain segment as a structure condition to obtain a candidate segment. That is, the molecule is separated, and a construction rule of a relevant segment is learned. The fragment segment on the original molecule is replaced with the newly generated candidate segment so as to implement the modification of the molecule. A relatively large number of candidate segments may be generated according to the structure condition, so that the probability that a newly generated molecule is different from an existing molecular structure is increased, and furthermore, the success rate of molecular reconstruction is increased.

According to the method provided in this embodiment, candidate segments are screened to filter out invalid or repeated segments and filter out, according to a heavy atom number requirement, segments that do not meet the heavy atom number requirement. By step-by-step screening, the segments that meet the base requirements in the candidate segments are retained, and the segments that do not meet the requirements are deleted. Therefore, the problem of low molecular reconstruction efficiency caused by the fact that an excessive number of reconstructed molecules are subsequently generated and the molecules containing invalid segments therein may not be filtered is solved.

According to the method provided in this embodiment, 3D similarities between the candidate segments and the fragment segment are calculated to ensure that retained candidate segments are as close as possible to the fragment segment to be modified in the reference molecule in terms of 3D structure, so that the activity is easily maintained.

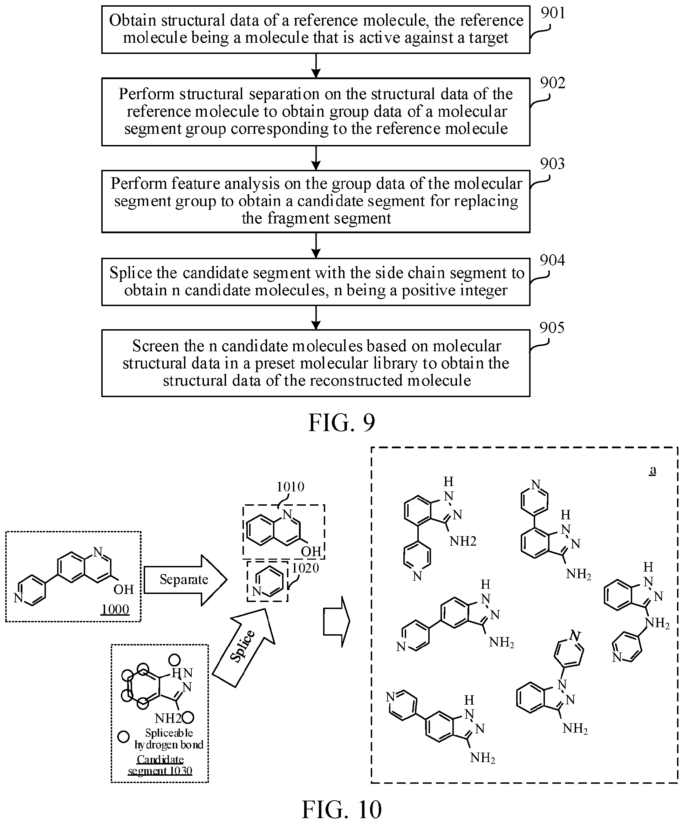

In some embodiments, molecular screening is further needed when the reconstructed molecule is generated based on the candidate segment and the side chain segment. FIG. 9 is a flowchart of a molecular structure reconstruction method according to another exemplary embodiment of this application. The method is described taking execution by a computer device as an example. As shown in FIG. 9, the method includes the following steps:

Step 901: Obtain structural data of a reference molecule, the reference molecule being a molecule that is active against a target.

In this embodiment, taking the field of medicine as an example, the reference molecule is a molecule for a target. That is, the reference molecule is active against the target, so as to generate a drug action at the target.

Step 902: Perform structural separation on the structural data of the reference molecule to obtain group data of at least one molecular segment group corresponding to the reference molecule.

In some embodiments, structural separation needs to be performed on the reference molecule based on a preset separation rule.

That is, a preset separation rule is obtained first, the preset separation rule including at least one of a scaffold separation rule and a rotatable bond separation rule. A separation manner corresponding to the preset separation rule has been described in detail in step 302, and will not be elaborated herein.

Step 903: Perform feature analysis on the group data of the molecular segment group to obtain a candidate segment for replacing the fragment segment.

After the molecular segment group is input to a molecular reconstruction model, an encoder in the molecular reconstruction model encodes a fragment segment in the molecular segment group taking a side chain segment as a structure condition to obtain an encoded vector. Then, a decoder in the molecular reconstruction model decodes the encoded vector taking the side chain segment as a structure condition to obtain a candidate segment for replacing the fragment segment.

In this embodiment, descriptions are made taking the candidate segment as an example. In some embodiments, the candidate segment may also be implemented as a remaining segment after screening in combination with the solution provided in the embodiment shown in FIG. 8. That is, the candidate segment is screened first, and then the remaining segment is spliced with the subsequent side chain segment. No limits are made thereto in this embodiment of this application.

Step 904: Splice the candidate segment with the side chain segment to obtain n candidate molecules, n being a positive integer.

In some embodiments, after a portion to be modified (i.e., an original scaffold) in the reference molecule is deleted, a portion originally connected with the scaffold is docked with any H atom position on the candidate segment, with the SMILES validity thereof ensured, so as to generate new molecules, i.e., the foregoing n candidate molecules.

Schematically, referring to FIG. 10, FIG. 10 is a schematic diagram of a molecular site splicing manner according to an exemplary embodiment of this application. As shown in FIG. 10, a reference molecule 1000 is separated to obtain a fragment segment 1010 and a side chain segment 1020. After a candidate segment 1030 is generated through a molecular reconstruction model, the candidate segment 1030 is spliced with the side chain segment 1020 to obtain a splicing result 1040, including splicing results of different spliceable hydrogen bonds in the side chain segment 1020 and the candidate segment 1030.

Step 905: Screen the n candidate molecules based on molecular structural data in a preset molecular library to obtain structural data of a reconstructed molecule.

The reconstructed molecule is a newly generated molecule active against the target.

In some embodiments, the n candidate molecules are screened based on the molecular structural data in the preset molecular library to obtain m candidate molecules the preset molecular library does not include, $0 \leq m \leq n$. Then, the structural data of the reconstructed molecule is determined from the m candidate molecules.

In some embodiments, the spliced candidate molecules are screened with a purpose of avoiding existing molecules in the preset molecular library to remove molecules in the preset molecular library. Optionally, the preset molecular library includes a patent library or a patent scaffold collection. In some embodiments, the preset molecular library may be a molecular library set for the target. A screening manner is not limited in this embodiment of this application.

In some embodiments, a screening process of the candidate molecules further includes a pharmacological property screening process carried out by the computer device. That is, the generated candidate molecules are screened based on pharmacological properties. Schematically, the screening process specifically includes: 1: a mini capsule filter (MCF) that mainly removes candidate molecules containing reactive or toxic groups; and 2: physicochemical properties of the candidate molecules, for example: the candidate molecules need to satisfy the following conditions: the molecular weight (MW) is less than or equal to 550, the water solubility is within the range of [-5, 6], the topologically polar surface area of the molecule is less than or equal to 120, the number of rotatable bonds is less than 10, the number of hydrogen bond acceptors is within the range of [0, 10], the number of hydrogen bond donors is within the range of [0, 5], etc.

In some embodiments, when the reconstructed molecule is determined from the m candidate molecules, m molecular similarities between the m candidate molecules and the reference molecule respectively are obtained, an $i^{th}$

candidate molecule corresponding to an $i^{th}$ molecular similarity, and i being a positive integer. The structural data of the reconstructed molecule is determined from k candidate molecules with the maximum molecular similarity, $0 < k < m$.

That is, after the filtered candidate molecules are obtained, in order to ensure a higher probability of screening out an active molecule, the candidate molecule is required to be as close as possible to the reference molecule, to maintain the activity more easily. Therefore, 3D similarities between the candidate molecules and the reference molecule are calculated using RDKit or other 3D similarity calculation tools, and k candidate molecules with the maximum similarity are extracted for the subsequent process of generating the reconstructed molecule.

In some embodiments, when the candidate molecules are screened, key forces of the generated candidate molecules may be extracted using protein pocket information to screen out molecules that are not subjected to a force of the reference molecule. Then, by use of a molecular activity prediction model, the protein pocket information and the generated candidate molecules are input, and PIC50 activity values are output to sequence the candidate molecules, so as to achieve the purpose of screening.

In summary, according to the molecular structure reconstruction method provided in this embodiment of this application, structural separation is performed on a reference molecule to obtain a fragment segment and a side chain segment, so as to predict a new molecular segment structure replacing the fragment segment taking the side chain segment as a structure condition to obtain a candidate segment. That is, the molecule is separated according to a ring structure, and a construction rule of a relevant segment is learned. The fragment segment on the original molecule is replaced with the newly generated candidate segment so as to implement the modification of the molecule. A generated molecule may get out of a structural rule of an existing molecular structure at a high probability. Therefore, the success rate of molecular reconstruction is increased.

According to the method provided in this embodiment, after candidate molecules are generated, the candidate molecules are screened through a multilayer screening mechanism, so as to determine a reconstructed molecule from the candidate molecules after screening. Therefore, the problem of complicated artificial screening process caused by determining the reconstructed molecule from a large number of candidate molecules is solved.

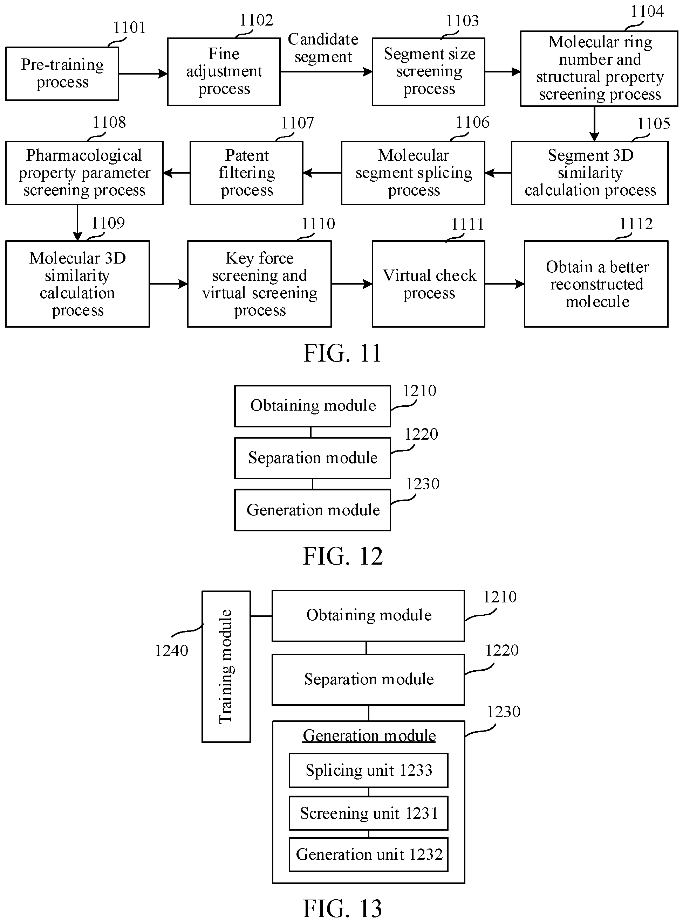

Schematically, FIG. 11 is an overall flowchart of a molecular structure reconstruction method according to an exemplary embodiment of this application. As shown in FIG. 11, the process includes the following steps:
Step 1101: Perform a pre-training process.

The pre-training process refers to learning molecules in a public database by a structure conditional variational autoencoder model, so that the model may learn a vector expression of a molecule better. The public database records drug-like molecules in a SMILES format.

In the pre-training process, a sample molecule is separated by at least one of two separation methods to obtain a scaffold and side chain pair. The scaffold and side chain pair obtained by separation is input to the structure conditional variational autoencoder model as training data, so as to complete a model pre-training task.

Step 1102: Perform a fine adjustment process.

In the fine adjustment process, a sample molecule active against a target is input and separated, and the model is further trained (a training manner is the same as that in the pre-training process, and an input dataset changes from the public database to a molecular dataset active against the target), so that a generated molecule is more likely to be a molecule similar to the molecule active against the target.

After the fine adjustment process ends, the model obtained by training may perform segment prediction on a reference molecule to obtain a candidate segment.

Step 1103: Perform a segment size screening process.

Optionally, SMILES validity and uniqueness processing is performed on a generated candidate segment set to remove invalid and repeated segments. Then, generated candidate segments are screened according to a selected heavy atom range to remove the candidate segments beyond the heavy atom range.

Step 1104: Perform a molecular ring number and structural property screening process.

Candidate molecules are screened according to molecular ring numbers or aromatic ring numbers. Alternatively, candidate molecules are screened according to structural properties of the molecules.

Steps 1103 and 1104 are two parallel steps. 1103 may be performed prior to 1104. Alternatively, 1103 and 1104 may be performed at the same time.

Step 1105: Perform a segment 3D similarity calculation process.

After a large number of candidate segments are obtained, the candidate segments are required to be as close as to a portion to be modified in the reference molecule, to maintain the activity more easily. Therefore, 3D similarities between the generated segments and a reference segment are calculated using RDKit, and q candidate segments with the maximum similarity are extracted for further splicing.

Step 1106: Perform a molecular segment splicing process.

After the portion to be modified (original scaffold) in the reference molecule is deleted, a portion originally connected with the original scaffold is docked with any H atom position on the candidate segment, with the SMILES validity thereof ensured, so as to generate a new molecule.

Step 1107: Perform a preset molecular library filtering process.

The spliced candidate molecules are screened in a manner of avoiding a preset molecular library to remove molecules the preset molecular library includes.

Step 1108: Perform a pharmacological property parameter screening process.

The generated candidate molecules are screened based on pharmacological properties. Schematically, screening details specifically include: 1: a mini capsule filter (MCF) that mainly removes candidate molecules containing reactive or toxic groups; and 2: physicochemical properties of the candidate molecules, for example: the candidate molecules need to satisfy the following conditions: the MW is less than or equal to 550, the water solubility is within the range of [-5, 6], the topologically polar surface area of the molecule is less than or equal to 120, the number of rotatable bonds is less than 11, the number of hydrogen bond acceptors is within the range of [0, 11], the number of hydrogen bond donors is within the range of [0, 5], etc.

Steps 1107 and 1108 are two parallel steps.

Step 1109: Perform a molecular 3D similarity calculation process.

After the filtered candidate molecules are obtained, in order to ensure a higher probability of screening out an active molecule, the candidate molecule is required to be as close as possible to the reference molecule, to maintain the activity more easily. 3D similarities between the candidate molecules and the reference molecule are calculated using RDKit or other 3D similarity calculation tools, and top k candidate molecules are extracted for the subsequent process of generating the reconstructed molecule.

Step 1110: Perform a key force screening and virtual screening process.

When the candidate molecules are screened, key forces of the generated candidate molecules may be extracted using protein pocket information to screen out molecules that are not subjected to a force of the reference molecule. Then, by use of a molecular activity prediction model, the protein pocket information and the generated candidate molecules are input, and PIC50 activity values are output to sequence the candidate molecules, so as to achieve the purpose of screening.

Step 1111: Perform a virtual check process.

In some embodiments, the candidate molecules remaining after layer-by-layer screening are manually screened.

Step 1112: Obtain a better reconstructed molecule.

In general, dozens of candidate molecules are screened from the candidate molecules as better molecules.

In summary, according to the molecular structure reconstruction method provided in this embodiment of this application, structural separation is performed on a reference molecule to obtain a fragment segment and a side chain segment, so as to predict a new molecular segment structure replacing the fragment segment taking the side chain segment as a structure condition to obtain a candidate segment. That is, the molecule is separated, and a construction rule of a relevant segment is learned. The fragment segment on the original molecule is replaced with the newly generated candidate segment so as to implement the modification of the molecule. A relatively large number of candidate segments may be generated according to the structure condition, so that the probability that a newly generated molecule is different from an existing molecular structure is increased, and furthermore, the success rate of molecular reconstruction is increased.

FIG. 12 is a schematic structural diagram of a molecular structure reconstruction apparatus according to an exemplary embodiment of this application. As shown in FIG. 12, the apparatus includes:

an obtaining module 1210, configured to obtain a set of structural data of a reference molecule, the reference molecule being a molecule that is active against a pre-set target;

a separation module 1220, configured to structural separation on the obtained set of structural data to obtain a plurality of subsets of group data each corresponding to one of a plurality of molecular segment groups of the reference molecule, the molecular segment groups including each a fragment segment of the reference molecule and a side chain segment corresponding to the fragment segment; and

a generation module 1230, configured to perform feature analysis on the group data of the molecular segment group to obtain a candidate segment for replacing the fragment segment,

the generation module 1230 being further configured to generate structural data of a reconstructed molecule based on the candidate segment and the side chain segment, the reconstructed molecule being active against the target.

In an optional embodiment, the obtaining module 1210 is further configured to obtain a preset separation rule, the preset separation rule including at least one of a scaffold separation rule and a rotatable bond separation rule.

The separation module 1220 is further configured to perform structural separation on the structural data of the reference molecule based on the preset separation rule to obtain group data of at least one molecular segment group corresponding to the reference molecule.

In an optional embodiment, the preset separation rule includes the scaffold separation rule.

The separation module 1220 is further configured to extract a scaffold structure meeting a scaffold requirement from the structural data of the reference molecule, delete the scaffold structure from the structural data of the reference molecule to obtain a side chain structure corresponding to the scaffold structure, and obtain the group data of the at least one molecular segment group corresponding to the reference molecule taking the scaffold structure as the fragment segment and the side chain structure as the side chain segment.

In an optional embodiment, the separation module 1220 is further configured to extract a primary scaffold structure from the structural data of the reference molecule, the primary scaffold structure being a largest scaffold structure in the reference molecule, extract a secondary scaffold structure from the primary scaffold structure, and determine the scaffold structure meeting the scaffold requirement from the primary scaffold structure and the secondary scaffold structure.

In an optional embodiment, the scaffold requirement includes at least one of a ring number requirement, a heavy atom number requirement, and a rotatable bond number requirement.

The separation module 1220 is further configured to extract the scaffold structure whose ring number is within a range of the ring number requirement from the structural data of the reference molecule in response to the scaffold requirement including the ring number requirement.

The separation module 1220 is further configured to extract the scaffold structure whose heavy atom number is within a range of the heavy atom number requirement from the structural data of the reference molecule in response to the scaffold requirement including the heavy atom number requirement.

The separation module 1220 is further configured to extract the scaffold structure whose rotatable bond number is within a range of the rotatable bond number requirement from the structural data of the reference molecule in response to the scaffold requirement including the rotatable bond number requirement.

In an optional embodiment, the preset separation rule includes the rotatable bond separation rule.

The separation module 1220 is further configured to cleave the structural data of the reference molecule from a rotatable bond to obtain the fragment segment meeting a fragment requirement, delete the fragment segment from the reference molecule to obtain the side chain segment, and obtain the group data of the at least one molecular segment group corresponding to the reference molecule based on the fragment segment and the side chain segment.

In an optional embodiment, each fragment segment includes a fragment scaffold, and the fragment requirement includes at least one of a fragment base requirement and a fragment scaffold requirement.

The fragment base requirement includes at least one of a fragment ring number requirement, a fragment atom number requirement, a fragment rotatable bond number requirement, and a fragment structure requirement.

The fragment scaffold requirement includes at least one of a scaffold atom number requirement and scaffold rotatable bond number requirement for the fragment scaffold.

In an optional embodiment, as shown in FIG. 13, the generation module 1230 includes:

a screening unit 1231, configured to screen the candidate segment based on a preset screening rule, the preset screening rule including at least one of a validity screening rule, a uniqueness screening rule, a heavy atom number screening rule, and a ring screening rule; and

a generation unit 1232, configured to generate the structural data of the reconstructed molecule based on a remaining segment after screening and the side chain segment.

In an optional embodiment, the obtaining module 1210 is further configured to obtain a segment similarity between the remaining segment and the fragment segment.

The generation unit 1232 is further configured to retain $q$ remaining segments with a maximum segment similarity, and generate the structural data of the reconstructed molecule together with the side chain segment, $q$ being a positive integer.

In an optional embodiment, the generation module 1230 is further configured to input the group data of the molecular segment group to a molecular reconstruction model, and perform feature analysis on the group data of the molecular segment group through the molecular reconstruction model to output the candidate segment for replacing the fragment segment.

In an optional embodiment, the molecular reconstruction model includes an encoder and decoder in an encoding and decoding architecture.

The generation module 1230 is further configured to encode the fragment segment through the encoder based on the side chain segment to obtain a segment feature, apply a perturbation to the segment feature through a preset perturbation rule to obtain a perturbed feature, and decode the perturbed feature through the decoder to generate the candidate segment for replacing the fragment segment.

In an optional embodiment, the generation module 1230 is further configured to map the side chain segment to a feature space to obtain a side chain spatial feature, and encode the fragment segment through the encoder taking the side chain spatial feature as a structure condition to obtain the segment feature.

In an optional embodiment, the generation module 1230 is further configured to encode the fragment segment to obtain an encoded feature, and map the encoded feature to the feature space taking the side chain spatial feature as the structure condition to obtain the segment feature.

In an optional embodiment, the molecular reconstruction model includes a model parameter.

The obtaining module 1210 is further configured to obtain structural data of a sample molecule, the sample molecule being separable into at least one group of sample fragment and sample side chain.

The apparatus further includes:

a training module 1240, configured to perform feature analysis on the structural data of the sample molecule through the molecular reconstruction model to output a reconstructed fragment for replacing the sample fragment, and adjusting the model parameter based on a difference degree between the sample fragment and the reconstructed fragment.

In an optional embodiment, the training module 1240 is further configured to adjust the model parameter based on a discrete degree of mapping of the sample fragment and the reconstructed fragment in a feature space, the feature space being determined by the model parameter.

In an optional embodiment, the generation module 1230 includes:

a splicing unit 1233, configured to splice a hydrogen atom position on the candidate segment with the side chain segment to obtain n candidate molecules, n being a positive integer; and

a screening unit 1231, configured to screen the n candidate molecules based on molecular structural data in a preset molecular library to obtain the structural data of the reconstructed molecule.

In an optional embodiment, the screening unit 1231 is further configured to screen the n candidate molecules based on the molecular structural data in the preset molecular library to obtain m candidate molecules the preset molecular library does not include, $0 \leq m \leq n$.

The generation module 1230 further includes:

a generation unit 1232, configured to determine the structural data of the reconstructed molecule from the m candidate molecules.

In an optional embodiment, the generation unit 1232 is further configured to: obtain m molecular similarities between the m candidate molecules and the reference molecule respectively, an $i^{th}$ candidate molecule corresponding to an $i^{th}$ molecular similarity, and i being a positive integer; and determine the structural data of the reconstructed molecule from k candidate molecules with the maximum molecular similarity, $0 < k < m$.

In summary, according to the molecular structure reconstruction apparatus provided in this embodiment of this application, structural separation is performed on structural data of a reference molecule to obtain a fragment segment and a side chain segment, so as to predict a new molecular segment structure replacing the fragment segment taking the side chain segment as a structure condition to obtain a candidate segment. That is, the molecule is separated, and a construction rule of a relevant segment is learned. The fragment segment on the original molecule is replaced with the newly generated candidate segment so as to implement the modification of the molecule. A relatively large number of candidate segments may be generated according to the structure condition, so that the probability that a newly generated molecule is different from an existing molecular structure is increased, and furthermore, the success rate of molecular reconstruction is increased.

It is to be noted that the molecular structure reconstruction apparatus provided in the foregoing embodiment is illustrated with an example of division of the foregoing functional modules. In actual application, the functions may be allocated to and completed by different functional modules according to requirements. That is, the internal structure of the device is divided into different functional modules, to implement all or some of the functions described above. In addition, the molecular structure reconstruction apparatus provided in the foregoing embodiments and the embodiments of the molecular structure reconstruction method belong to the same conception. For the specific implementation process, reference may be made to the method embodiments, and details are not described herein again.

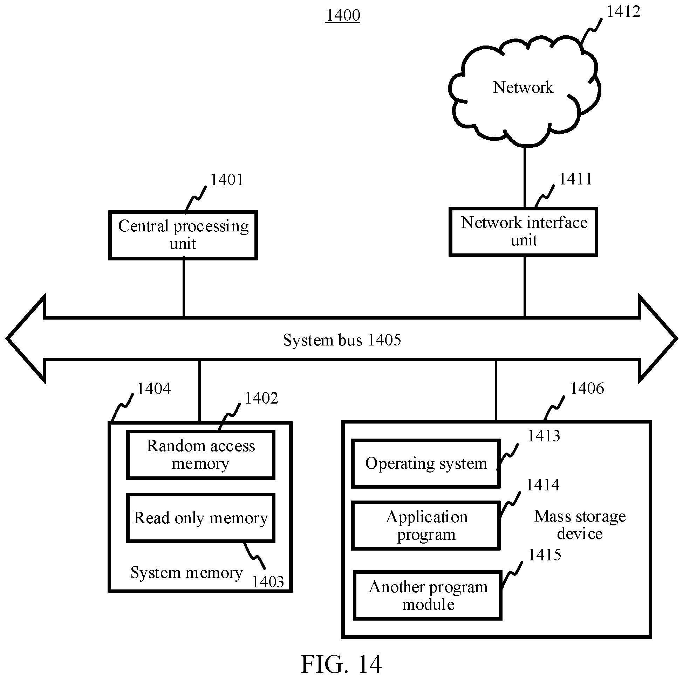

FIG. 14 is a structural schematic diagram of a computer device according to an exemplary embodiment of this application. The computer device may be implemented as a server or a terminal. Specifically,

the computer device 1400 includes a central processing unit (CPU) 1401, a system memory 1404 including a random access memory (RAM) 1402 and a read only memory (ROM) 1403, and a system bus 1405 connecting the system memory 1404 to the CPU 1401. The computer device 1400 further includes a mass storage device 1406 configured to store an operating system 1413, an application program 1414, and another program module 1415.

The mass storage device 1406 is connected to the CPU 1401 by using a mass storage controller (not shown) connected to the system bus 1405. The mass storage device 1406 and a computer-readable medium associated with the mass storage device provide non-volatile storage to the computer device 1400. That is, the mass storage device 1406 may include a computer-readable medium (not shown) such as a hard disk, or a compact disc read-only memory (CD- ROM) drive.

Without loss of generality, the computer-readable medium may include a computer storage medium and a communication medium. The computer storage medium includes volatile and non-volatile media, and removable and non-removable media implemented by using any method or technology configured to store information such as a computer-readable instruction, a data structure, a program module, or other data. The computer storage medium includes a RAM, a ROM, an erasable programmable ROM (EPROM), an electrically erasable programmable ROM (EEPROM), a flash memory or another solid-state memory technology, a CD-ROM, a digital versatile disc (DVD) or another optical memory, a tape cartridge, a magnetic cassette, a magnetic disk memory, or another magnetic storage device. Certainly, a person skilled in the art can know that the computer storage medium is not limited to the foregoing several types. The foregoing system memory 1404 and the mass storage device 1406 may be collectively referred to as a memory.

According to the embodiments of this application, the computer device 1400 may further be connected, through a network such as the Internet, to a remote computer on the network and run. That is, the computer device 1400 may be connected to a network 1412 by using a network interface unit 1411 connected to the system bus 1405, or may be connected to another type of network or a remote computer system (not shown) by using a network interface unit 1411.

The memory further includes one or more programs, which are stored in the memory and are configured to be executed by the CPU.

This embodiment of this application further provides a computer device, including processor and a memory, the memory storing at least one instruction, at least one program, a code set, or an instruction set, the at least one instruction, the at least one program, the code set, or the instruction set being loaded and executed by the processor to implement the molecular structure reconstruction method provided in the foregoing method embodiments.

This embodiment of this application further provides a computer-readable storage medium, storing at least one instruction, at least one program, a code set, or an instruction set, the at least one instruction, the at least one program, the code set or the instruction set being loaded and executed by a processor to implement the molecular structure reconstruction method provided in the foregoing method embodiments.

This embodiment of this application further provides a computer program product or a computer program, including computer instructions, the computer instructions being stored in a computer-readable storage medium. A processor of a computer device reads the computer instructions from the computer-readable storage medium and executes the computer instructions to cause the computer device to perform the molecular structure reconstruction method according to any one of the embodiments.

**Claims**

1. A molecular structure reconstruction method, applied to a computer device, the method comprising:

   obtaining a set of structural data of a reference molecule, the reference molecule being a molecule that is active against a pre-set target;
   performing structural separation on the obtained set of structural data to obtain a plurality of subsets of group data each corresponding to one of a plurality of molecular segment groups of the reference molecule, the molecular segment groups comprising each a fragment segment of the reference molecule and a side chain segment corresponding to the fragment segment;
   performing feature analysis on the group data of the molecular segment group to obtain a candidate segment for replacing the fragment segment; and
   generating structural data of a reconstructed molecule based on the candidate segment and the side chain segment, the reconstructed molecule being active against the target.

2. The method according to claim 1, wherein the performing structural separation on the obtained set of structural data to obtain a plurality of subsets of group data each corresponding to one of a plurality of molecular segment groups of the reference molecule comprises:

   obtaining a preset separation rule, the preset separation rule comprising at least one of a scaffold separation rule and a rotatable bond separation rule; and
   performing structural separation on the structural data of the reference molecule based on the preset separation rule to obtain group data of at least one molecular segment group corresponding to the reference molecule.

3. The method according to claim 2, wherein the preset separation rule comprises the scaffold separation rule;
the performing structural separation on the structural data of the reference molecule based on the preset separation rule to obtain group data of at least one molecular segment group corresponding to the reference molecule comprises:

extracting a scaffold structure meeting a scaffold requirement from the structural data of the reference molecule;
deleting the scaffold structure from the structural data of the reference molecule to obtain a side chain structure corresponding to the scaffold structure; and
obtaining the group data of the at least one molecular segment group corresponding to the reference molecule taking the scaffold structure as the fragment segment and the side chain structure as the side chain segment.

4. The method according to claim 3, wherein the extracting a scaffold structure meeting a scaffold requirement from the reference molecule comprises:

extracting a primary scaffold structure from the structural data of the reference molecule, the primary scaffold structure being a largest scaffold structure in the reference molecule;
extracting a secondary scaffold structure from the primary scaffold structure; and
determining the scaffold structure meeting the scaffold requirement from the primary scaffold structure and the secondary scaffold structure.

5. The method according to claim 3, wherein the scaffold requirement comprises at least one of a ring number requirement, a heavy atom number requirement, and a rotatable bond number requirement;
the extracting a scaffold structure meeting a scaffold requirement from the reference molecule comprises:

extracting the scaffold structure whose ring number is within a range of the ring number requirement from the structural data of the reference molecule in response to the scaffold requirement comprising the ring number requirement;
extracting the scaffold structure whose heavy atom number is within a range of the heavy atom number requirement from the structural data of the reference molecule in response to the scaffold requirement comprising the heavy atom number requirement; and
extracting the scaffold structure whose rotatable bond number is within a range of the rotatable bond number requirement from the structural data of the reference molecule in response to the scaffold requirement comprising the rotatable bond number requirement.

6. The method according to claim 2, wherein the preset separation rule comprises the rotatable bond separation rule;
the performing structural separation on the structural data of the reference molecule based on the preset separation rule to obtain group data of at least one molecular segment group corresponding to the reference molecule comprises:

cleaving the structural data of the reference molecule from a rotatable bond to obtain the fragment segment meeting a fragment requirement;
deleting the fragment segment from the reference molecule to obtain the side chain segment; and
obtaining the group data of the at least one molecular segment group corresponding to the reference molecule based on the fragment segment and the side chain segment.

7. The method according to claim 6, wherein each fragment segment comprises a fragment scaffold, and the fragment requirement comprises at least one of a fragment base requirement and a fragment scaffold requirement;

the fragment base requirement comprises at least one of a fragment ring number requirement, a fragment atom number requirement, a fragment rotatable bond number requirement, and a fragment structure requirement; and
the fragment scaffold requirement comprises at least one of a scaffold atom number requirement and scaffold rotatable bond number requirement for the fragment scaffold.

8. The method according to any one of claims 1-7, wherein the performing feature analysis on the molecular segment group to obtain a candidate segment for replacing the fragment segment comprises:

inputting the group data of the molecular segment group to a molecular reconstruction model; and
performing feature analysis on the group data of the molecular segment group through the molecular reconstruction model to output the candidate segment for replacing the fragment segment.

9. The method according to claim 8, wherein the molecular reconstruction model comprises an encoder and decoder in an encoding and decoding architecture;
the performing feature analysis on the group data of the molecular segment group through the molecular reconstruction model to output the candidate segment for replacing the fragment segment comprises:

encoding the fragment segment through the encoder based on the side chain segment to obtain a segment feature;
applying a perturbation to the segment feature through a preset perturbation rule to obtain a perturbed feature; and
decoding the perturbed feature through the decoder to generate the candidate segment for replacing the fragment segment.

10. The method according to claim 9, wherein the encoding the fragment segment through the encoder based on the side chain segment to obtain a segment feature comprises:

mapping the side chain segment to a feature space to obtain a side chain spatial feature; and
encoding the fragment segment through the encoder taking the side chain spatial feature as a structure condition to obtain the segment feature.

11. The method according to claim 10, wherein the encoding the fragment segment through the encoder taking the side chain spatial feature as a structure condition to obtain the segment feature comprises:

encoding the fragment segment to obtain an encoded feature; and
mapping the encoded feature to the feature space taking the side chain spatial feature as the structure condition to obtain the segment feature.

12. The method according to claim 8, wherein a training process of the molecular reconstruction model comprises:

obtaining structural data of a sample molecule, the sample molecule being separable into at least one group of sample fragment and sample side chain;
performing feature analysis on the structural data of the sample molecule through the molecular reconstruction model to output a reconstructed fragment for replacing the sample fragment; and
adjusting a model parameter of the molecular reconstruction model based on a difference degree between the sample fragment and the reconstructed fragment.

13. The method according to claim 12, wherein the adjusting a model parameter based on a difference degree between the sample fragment and the reconstructed fragment comprises:
adjusting the model parameter based on a discrete degree of mapping of the sample fragment and the reconstructed fragment in a feature space, the feature space being determined by the model parameter.

14. The method according to any one of claims 1-7, wherein the generating structural data of a reconstructed molecule based on the candidate segment and the side chain segment comprises:

screening the candidate segment based on a preset screening rule, the preset screening rule comprising at least one of a validity screening rule, a uniqueness screening rule, a heavy atom number screening rule, and a ring screening rule; and
generating the structural data of the reconstructed molecule based on a remaining segment after screening and the side chain segment.

15. The method according to claim 14, wherein the generating the structural data of the reconstructed molecule based on a remaining segment after screening and the side chain segment comprises:

obtaining a segment similarity between the remaining segment and the fragment segment; and
retaining q remaining segments with a maximum segment similarity, and generating the structural data of the reconstructed molecule together with the side chain segment, q being a positive integer.

16. The method according to any one of claims 1-7, wherein the generating structural data of a reconstructed molecule based on the candidate segment and the side chain segment comprises:

splicing a hydrogen atom position on the candidate segment with the side chain segment to obtain n candidate molecules, n being a positive integer; and

screening the n candidate molecules based on molecular structural data in a preset molecular library to obtain the structural data of the reconstructed molecule.

17. The method according to claim 16, wherein the screening the n candidate molecules based on molecular structural data in a preset molecular library to obtain the structural data of the reconstructed molecule comprises:

screening the n candidate molecules based on the molecular structural data in the preset molecular library to obtain m candidate molecules the preset molecular library does not comprise, $0 \leq m \leq n$; and

determining the structural data of the reconstructed molecule from the m candidate molecules.

18. The method according to claim 17, wherein the determining the structural data of the reconstructed molecule from the m candidate molecules comprises:

obtaining m molecular similarities between the m candidate molecules and the reference molecule respectively, an $i^{th}$ candidate molecule corresponding to an $i^{th}$ molecular similarity, and i being a positive integer; and

determining the structural data of the reconstructed molecule from k candidate molecules with the maximum molecular similarity, $0 < k < m$.

19. A molecular structure reconstruction apparatus, comprising:

an obtaining module, configured to obtain a set of structural data of a reference molecule, the reference molecule being a molecule that is active against a pre-set target;

a separation module, configured to perform structural separation on the obtained set of structural data to obtain a plurality of subsets of group data each corresponding to one of a plurality of molecular segment groups of the reference molecule,, the molecular segment groups comprising each a fragment segment of the reference molecule and a side chain segment corresponding to the fragment segment; and

a generation module, configured to perform feature analysis on the group data of the molecular segment group to obtain a candidate segment for replacing the fragment segment,

the generation module being further configured to generate structural data of a reconstructed molecule based on the candidate segment and the side chain segment, the reconstructed molecule being active against the target.

20. The apparatus according to claim 19, wherein the obtaining module is further configured to obtain a preset separation rule, the preset separation rule comprising at least one of a scaffold separation rule and a rotatable bond separation rule; and

the separation module is further configured to perform structural separation on the structural data of the reference molecule based on the preset separation rule to obtain group data of at least one molecular segment group corresponding to the reference molecule.

21. The apparatus according to claim 20, wherein the preset separation rule comprises the scaffold separation rule; and the separation module is further configured to extract a scaffold structure meeting a scaffold requirement from the structural data of the reference molecule, delete the scaffold structure from the structural data of the reference molecule to obtain a side chain structure corresponding to the scaffold structure, and obtain the group data of the at least one molecular segment group corresponding to the reference molecule taking the scaffold structure as the fragment segment and the side chain structure as the side chain segment.

22. The apparatus according to claim 21, wherein the separation module is further configured to extract a primary scaffold structure from the structural data of the reference molecule, the primary scaffold structure being a largest scaffold structure in the reference molecule, extract a secondary scaffold structure from the primary scaffold structure, and determine the scaffold structure meeting the scaffold requirement from the primary scaffold structure and the secondary scaffold structure.

23. The apparatus according to claim 20, wherein the preset separation rule comprises the rotatable bond separation rule; and

the separation module is further configured to cleave the structural data of the reference molecule from a rotatable bond to obtain the fragment segment meeting a fragment requirement, delete the fragment segment from the reference molecule to obtain the side chain segment, and obtain the group data of the at least one molecular segment group corresponding to the reference molecule based on the fragment segment and the side chain segment.

24. A computer device, comprising a processor and a memory, the memory storing at least one program, the at least one program being loaded and executed by the processor to implement the molecular structure reconstruction method according to any one of claims 1 to 18.

25. A computer-readable storage medium, storing at least one program, the at least one program being loaded and executed by a processor to implement the molecular structure reconstruction method according to any one of claims 1 to 18.

26. A computer program product, comprising a computer program or an instruction, the computer program or the instruction, when executed by a processor, implementing the molecular structure reconstruction method according to any one of claims 1 to 18.

Obtain a set of structural data of a reference molecule, the reference molecule being a molecule that is active against a pre-set target — 101

Perform structural separation on the obtained set of structural data to obtain a plurality of subsets of group data each corresponding to one of a plurality of molecular segment groups of the reference molecule — 102

Perform feature analysis on the group data of the molecular segment group to obtain a candidate segment for replacing the fragment segment — 103

Generate structural data of a reconstructed molecule based on the candidate segment and the side chain segment — 104

FIG. 1

210

Molecule

220

Primary scaffold

230

Secondary scaffold

FIG. 2

FIG. 3

FIG. 4

FIG. 5

FIG. 6

FIG. 7

Obtain structural data of a reference molecule, the reference molecule being a molecule that is active against a target — 801

Perform structural separation on the structural data of the reference molecule to obtain group data of a molecular segment group corresponding to the reference molecule — 802

Perform feature analysis on the group data of the molecular segment group to obtain a candidate segment for replacing the fragment segment — 803

Screen the candidate segment based on a preset screening rule — 804

Generate the structural data of the reconstructed molecule based on a remaining segment after screening and the side chain segment — 805

FIG. 8

```
┌─────────────────────────────────────────────────────────┐  901
│ Obtain structural data of a reference molecule, the reference │
│   molecule being a molecule that is active against a target   │
└─────────────────────────────────────────────────────────┘
                            │
                            ▼
┌─────────────────────────────────────────────────────────┐  902
│ Perform structural separation on the structural data of the  │
│    reference molecule to obtain group data of a molecular    │
│   segment group corresponding to the reference molecule      │
└─────────────────────────────────────────────────────────┘
                            │
                            ▼
┌─────────────────────────────────────────────────────────┐  903
│ Perform feature analysis on the group data of the molecular  │
│  segment group to obtain a candidate segment for replacing   │
│                    the fragment segment                      │
└─────────────────────────────────────────────────────────┘
                            │
                            ▼
┌─────────────────────────────────────────────────────────┐  904
│ Splice the candidate segment with the side chain segment to  │
│   obtain n candidate molecules, n being a positive integer   │
└─────────────────────────────────────────────────────────┘
                            │
                            ▼
┌─────────────────────────────────────────────────────────┐  905
│    Screen the n candidate molecules based on molecular       │
│  structural data in a preset molecular library to obtain the │
│        structural data of the reconstructed molecule         │
└─────────────────────────────────────────────────────────┘
```

FIG. 9

FIG. 10

1101

Pre-training process

1102

Fine adjustment process

Candidate segment

1103

Segment size screening process

1104

Molecular ring number and structural property screening process

1108

Pharmacological property parameter screening process

1107

Patent filtering process

1106

Molecular segment splicing process

1105

Segment 3D similarity calculation process

1109

Molecular 3D similarity calculation process

1110

Key force screening and virtual screening process

1111

Virtual check process

1112

Obtain a better reconstructed molecule

FIG. 11

Obtaining module 1210

Separation module 1220

Generation module 1230

FIG. 12

Training module 1240

Obtaining module 1210

Separation module 1220

Generation module 1230

Splicing unit 1233

Screening unit 1231

Generation unit 1232

FIG. 13

1400

1412

Network

1401

Central processing unit

1411

Network interface unit

System bus 1405

1404

1402

Random access memory

Read only memory

System memory 1403

1406

1413

Operating system

1414

Application program

Mass storage device

1415

Another program module

FIG. 14

EP 4 266 317 A1

## INTERNATIONAL SEARCH REPORT

| | International application No. |
|---|---|
| | **PCT/CN2022/078182** |

**A. CLASSIFICATION OF SUBJECT MATTER**

G16C 20/50(2019.01)i; G16C 20/70(2019.01)i

According to International Patent Classification (IPC) or to both national classification and IPC

**B. FIELDS SEARCHED**

Minimum documentation searched (classification system followed by classification symbols)

G16C

Documentation searched other than minimum documentation to the extent that such documents are included in the fields searched

Electronic data base consulted during the international search (name of data base and, where practicable, search terms used)

CNTXT, ENTXT, ENTXTC, DWPI, CNKI: 分子, 拆分, 分解, 片段, 替换, 候选, 重建, 生成, 优化, 合并, 组合, 编码, 解码; molecular, molecule, decompose, divide, fragment, replace, substitute, candidate, generate, optimize, combine, assemble, encoder, decoder.

**C. DOCUMENTS CONSIDERED TO BE RELEVANT**

| Category* | Citation of document, with indication, where appropriate, of the relevant passages | Relevant to claim No. |
|---|---|---|
| Y | US 2013226549 A1 (ZENG YUFENG) 29 August 2013 (2013-08-29) <br> description, paragraphs 0047-0115, and figures 1-4 | 1-26 |
| Y | CN 112116963 A (SHENZHEN ZHIYAO INFORMATION TECHNOLOGY CO., LTD.) 22 December 2020 (2020-12-22) <br> description, paragraphs 0031-0099, claims 1-6, and figures 2A-11 | 1-26 |
| A | US 2001056329 A1 (SMELLIE, A. S. et al.) 27 December 2001 (2001-12-27) <br> entire document | 1-26 |
| A | CN 111354424 A (BEIJING JINGPAI TECHNOLOGY CO., LTD.) 30 June 2020 (2020-06-30) <br> entire document | 1-26 |
| A | CN 109741798 A (SHENZHEN XTALPI TECHNOLOGY INC.) 10 May 2019 (2019-05-10) <br> entire document | 1-26 |

☐ Further documents are listed in the continuation of Box C.    ☑ See patent family annex.

| | |
|---|---|
| \*   Special categories of cited documents: <br> "A"   document defining the general state of the art which is not considered to be of particular relevance <br> "E"   earlier application or patent but published on or after the international filing date <br> "L"   document which may throw doubts on priority claim(s) or which is cited to establish the publication date of another citation or other special reason (as specified) <br> "O"   document referring to an oral disclosure, use, exhibition or other means <br> "P"   document published prior to the international filing date but later than the priority date claimed | "T"   later document published after the international filing date or priority date and not in conflict with the application but cited to understand the principle or theory underlying the invention <br> "X"   document of particular relevance; the claimed invention cannot be considered novel or cannot be considered to involve an inventive step when the document is taken alone <br> "Y"   document of particular relevance; the claimed invention cannot be considered to involve an inventive step when the document is combined with one or more other such documents, such combination being obvious to a person skilled in the art <br> "&"   document member of the same patent family |

| Date of the actual completion of the international search | Date of mailing of the international search report |
|---|---|
| **21 April 2022** | **26 April 2022** |

| Name and mailing address of the ISA/CN | Authorized officer |
|---|---|
| **China National Intellectual Property Administration (ISA/CN)** <br> **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2022/078182**

| Patent document cited in search report | | | Publication date (day/month/year) | Patent family member(s) | | | Publication date (day/month/year) |
|---|---|---|---|---|---|---|---|
| US | 2013226549 | A1 | 29 August 2013 | TW | 201408828 | A | 01 March 2014 |
| | | | | TW | I611053 | B | 11 January 2018 |
| CN | 112116963 | A | 22 December 2020 | None | | | |
| US | 2001056329 | A1 | 27 December 2001 | AU | 8161098 | A | 04 January 1999 |
| | | | | WO | 9859306 | A1 | 30 December 1998 |
| | | | | JP | 2002506447 | A | 26 February 2002 |
| | | | | EP | 0992011 | A1 | 12 April 2000 |
| | | | | CA | 2291070 | A1 | 30 December 1998 |
| | | | | DE | 69807485 | D1 | 02 October 2002 |
| | | | | NZ | 501068 | A | 25 October 2002 |
| | | | | AU | 751474 | B2 | 15 August 2002 |
| | | | | NZ | 517455 | A | 31 October 2003 |
| CN | 111354424 | A | 30 June 2020 | None | | | |
| CN | 109741798 | A | 10 May 2019 | WO | 2020029521 | A1 | 13 February 2020 |
| | | | | US | 2021065850 | A1 | 04 March 2021 |

Form PCT/ISA/210 (patent family annex) (January 2015)

**EP 4 266 317 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202110260462

30